# EUROPEAN PATENT APPLICATION

(11) **EP 4 201 312 A1**
(43) Date of publication of application: **28.06.2023**
(21) Application number: 22215060.9
(22) Date of filing: 20.12.2022
(51) Int. Cl.: A61B 5/00, A61B 10/02, A61B 17/34, A61B 1/018

(54) **PRESSURE SENSOR MODULE FOR AN INTERVENTIONAL MEDICAL DEVICE**

(30) Priority: 21.12.2021 US 202117557586
(71) Applicant: Creganna Unlimited Company, Ballybrit, Galway (IE); Measurement Specialties, Inc., Hampton, VA 23666 (US)
(72) Inventor: White, Annette Kay, Wilsonville, 97070-9289 (US); Woods, Jason, Wilsonville, 97070-9289 (US); Medina, Thomas, Portland, 97229 (US); Gaynor, Justin, Milpitas, 95035 (US); Wagner, Chris, San Jose, 95125 (US); Rezayee, Jamal, Wilsonville, 97070-9289 (US); Stankard, Edward, Galway, H91 VN2T (IE)
(74) Representative: Johnstone, Douglas Ian

(57) **Abstract**

An interventional medical device (100) including an insertion tube (120) extending between a proximal end (102) and a distal end (104). The distal end (104) is configured to be inserted into internal tissues of a patient during a medical procedure. The insertion tube (120) has an internal channel (122). The interventional medical device (100) includes a tool (108) received in the internal channel (122) of the insertion tube (120). The tool (108) has a tool body (140) and a needle (160) at an end of the tool body (140) forming a tip (162) of the tool (108). The tool body (140) forms a tool channel (148). The interventional medical device (100) includes a pressure sensor module (200) received in the tool channel (148). The pressure sensor module (200) includes a pressure sensor (202) and a carrier (206) holding the pressure sensor (202). The carrier (206) is coupled to the tool body (140) to position the pressure sensor (202) in the tool channel (148) proximate to the tip (162) of the tool (108) for measuring pressure of fluid of the internal tissues of the patient.

## Description

### BACKGROUND OF THE INVENTION

The subject matter herein relates generally to an interventional medical device.

Interventional medical devices, such as endoscopes, are used during medical procedures for performing medical procedures on a patient. An endoscope typically includes an elongated tube that may be inserted into the body of the patient. A video camera or a fiber optic lens is provided at the distal end for viewing the body tissues as the tube is moved through the patient's body. Various surgical tools may be inserted through the interior of the tube for performing surgical procedures. For example, some known endoscopes include needles for taking biopsies of tissue of a patient. Proper locating of the distal end of the endoscope is problematic. For example, it is difficult to locate the tip of the tool at the diseased tissue for biopsy. It is also difficult to determine when certain tissues or organs have been pierced. Current endoscopic procedures are time consuming due to the difficulty in locating the endoscope during the procedure.

A need remains for a reliable interventional medical device having improved diagnostics for performing medical procedures on patients.

### BRIEF DESCRIPTION OF THE INVENTION

In one embodiment, an interventional medical device is provided and includes an insertion tube extending between a proximal end and a distal end. The distal end configured to be inserted into internal tissues of a patient during a medical procedure. The insertion tube has an internal channel. The interventional medical device includes a tool received in the internal channel of the insertion tube. The tool has a tool body and a needle at an end of the tool body that forms a tip of the tool. The tool body forms a tool channel. The interventional medical device includes a pressure sensor module received in the tool channel. The pressure sensor module includes a pressure sensor and a carrier holding the pressure sensor. The carrier is coupled to the tool body to position the pressure sensor in the tool channel proximate to the tip of the tool for measuring pressure of fluid of the internal tissues of the patient.

In another embodiment, an interventional medical device is provided and includes a handle. The interventional medical device includes an insertion tube extending between a proximal end and a distal end. The proximal end extending from the handle. The distal end is configured to be inserted into internal tissues of a patient during a medical procedure. The insertion tube has an internal channel. The interventional medical device includes a light transmitter received in the internal channel of the insertion tube and extending to the distal end for illuminating the internal tissues of the patient. The interventional medical device includes a camera at the distal end for imaging the internal tissues of the patient. The camera has a cable extending from the camera through the internal channel. The interventional medical device includes a tool received in the internal channel of the insertion tube. The tool has a tool body and a needle at an end of the tool body that forms a tip of the tool. The tool body forms a tool channel. The interventional medical device includes a pressure sensor module received in the tool channel. The pressure sensor module includes a pressure sensor and a carrier holding the pressure sensor. The carrier is coupled to the tool body to position the pressure sensor in the tool channel proximate to the tip of the tool for measuring pressure of fluid of the internal tissues of the patient.

In a further embodiment, an interventional medical device is provided and includes an insertion tube extending between a proximal end and a distal end. The distal end is configured to be inserted into internal tissues of a patient during a medical procedure. The insertion tube has an internal channel. The interventional medical device includes a tool received in the internal channel of the insertion tube. The tool has a tool body and a needle at an end of the tool body that forms a tip of the tool. The tool body forms a tool channel. The interventional medical device includes a pressure sensor module received in the tool channel. The pressure sensor module includes a first pressure sensor and a second pressure sensor remote from the first pressure sensor. The first and second pressure sensors configured to measure pressure of fluid in the internal tissues. The pressure sensor module determining a first pressure from the first pressure sensor and determining a second pressure from the second pressure sensor.

### BRIEF DESCRIPTION OF THE DRAWINGS

Figure 1 is a perspective view of an interventional medical device in accordance with an exemplary embodiment.
Figure 2 is a perspective view of the tool in accordance with an exemplary embodiment.
Figure 3 is a perspective view of the pressure sensor module in accordance with an exemplary embodiment.
Figure 4 is a cross-sectional view of a portion of the tool in accordance with an exemplary embodiment.
Figure 5 is a perspective view of the tool in accordance with an exemplary embodiment.
Figure 6 is a perspective view of the pressure sensor module in accordance with an exemplary embodiment.
Figure 7 is a cross-sectional view of a portion of the tool in accordance with an exemplary embodiment.
Figure 8 is a perspective view of the tool in accordance with an exemplary embodiment.
Figure 9 is a perspective view of the pressure sensor module in accordance with an exemplary embodiment.

### DETAILED DESCRIPTION OF THE INVENTION

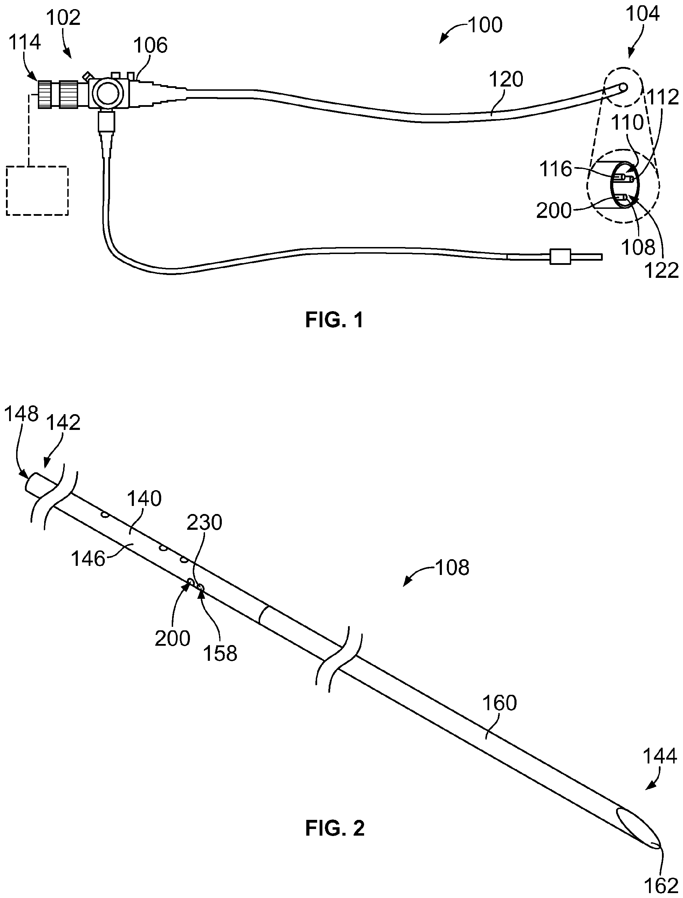

Figure 1 is a perspective view of an interventional medical device 100 in accordance with an exemplary embodiment. The interventional medical device 100 is used during a medical procedure for performing a medical procedure on a patient. In various embodiments, the interventional medical device 100 is an endoscope. However, the interventional medical device 100 may be another type of medical device, such as a a biopsy needle. In an exemplary embodiment, the interventional medical device 100 is configured to take pressure measurements of fluid in internal tissues of a patient during a medical procedure. The interventional medical device 100 may be used in a biopsy procedure. The interventional medical device 100 may be used in an ultrasound guided fine needle aspiration procedure.

The interventional medical device 100 extends between a proximal end 102 and a distal end 104. The interventional medical device 100 includes a handle 106 at the proximal end 102. The handle 106 may be used to actuate or manipulate the distal end 104 and/or one or more tools 108 at the distal end 104 for performing a medical procedure. For example, the distal end 104 may be steerable to position the tool 108 to perform the medical procedure, such as to biopsy damaged tissue. The handle 106 may be rotated and/or translated to actuate or manipulate the distal end 104, such as using pull wires, springs, or other types of actuation elements extending between the handle 106 and the distal end 104. In an exemplary embodiment, the tool 108 includes a pressure sensor module 200 at the distal end 104 to measure pressure of the fluid of the internal tissue. The pressure measurements may be used for locating the device relative to targeted tissues. The pressure measurements may be used to enhance tissue characterization with pressure measurements, such as to inform the clinician of disease state of the tissue to provide real time feedback to the clinician such as to better target sample extraction from a site. The pressure sensor module 200 includes electrical wires extending to the handle 106 or another device exterior of the interventional medical device 100, such as a monitor or computer. The pressure readout from the pressure sensor module 200 may be displayed or viewable at the exterior of the interventional medical device, such as at a remote computer monitor, at an attached display or dial, or at another location.

In an exemplary embodiment, the interventional medical device 100 includes an imaging system 110 for imaging the internal tissues of the patient. The imaging system 110 includes a camera 112 at the distal end 104 and a viewing device 114 at the proximal end 102. For example, the viewing device 114 may include an eyepiece coupled to the handle 106 in various embodiments, the viewing device 114 may include a display, such as a computer monitor showing the image. In an exemplary embodiment, the imaging system 110 includes a light transmitter 116 at the distal end 104 for illuminating the internal tissues. The light transmitter 116 may include one or more optical fibers and/or light pipes routed between the proximal end 102 and the distal end 104. The light transmitter 116 may include a lens at the distal end 104 that emits the light.

The interventional medical device 100 includes an insertion tube 120 extending between the proximal end 102 and the distal end 104. In an exemplary embodiment, the insertion tube 120 extends from the handle 106 at the proximal end 102 to the distal end 104. The distal end 104 of the insertion tube 120 is configured to be inserted into the patient and manipulated through the internal tissues of the body off the patient to a desired location (for example, a damaged tissue). The insertion tube 120 may be manufactured from medical grade material, such as a stainless steel material, a nitinol or nickel titanium material, or another suitable material. The insertion tube 120 may be coated, such as with a PTFE spray or jacket. The insertion tube 120 may include multiple sections. Portions of the insertion tube 120 may be rigid. Portions of the insertion tube 120 may be flexible to allow manipulation of the insertion tube 120 within the body of the patient.

In an exemplary embodiment, various components of the interventional medical device 100 are housed within the insertion tube 120 and extend between the handle 106 and the distal end 104. The insertion tube 120 includes an internal channel 122 that houses the components of the interventional medical device 100. For example, the tool 108 may be routed through the internal channel 122 to the distal end 104. The light transmitter 116 may be routed through the internal channel 122 to the distal end 104. The cameras 112 may be housed within the internal channel 122 at the distal end 104. Optionally, cables may extend from the camera 112 through the internal channel 122 to the handle 106 or another component, such as a computer to transmit the images from the camera 112 to the computer. Other components may be routed through the internal channel 122, such as air tubes, water tubes, electrical wires, and the like. For example, the electrical wires of the pressure sensor module 200 may be routed through the internal channel 122.

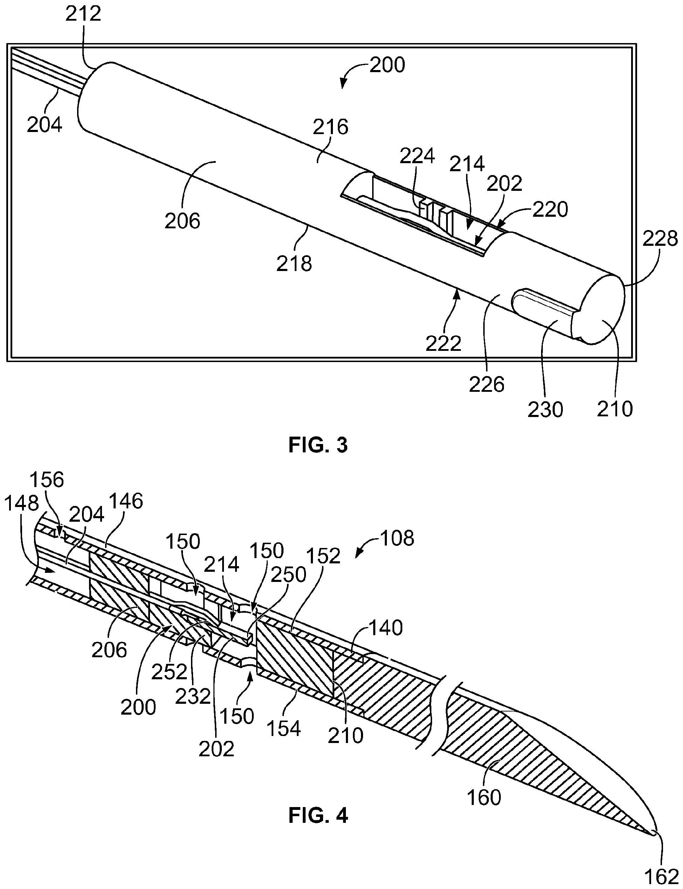

Figure 2 is a perspective view of the tool 108 in accordance with an exemplary embodiment. The tool 108 includes a tool body 140 extending between a first end 142 and a second end 144 of the tool 108. The second end 144 is a working end of the tool 108 that is used to interface with the internal tissues of the patient during the medical procedure. In an exemplary embodiment, the tool body 140 is generally cylindrical. However, the tool body 140 may have other shapes in alternative embodiments. The tool body 140 holds the pressure sensor module 200 (shown in Figure 3). The tool body 140 positions the pressure sensor module 200 proximate to the working end 144 to take pressure measurements proximate to the working end 144.

The tool body 140 includes a shaft 146. The shaft 146 may be hollow to receive components, such as electrical wires, control wires, and the like. In an exemplary embodiment, the pressure sensor module 200 is received in the hollow interior of the shaft 146. The shaft 146 may extend to the first end 142 of the tool 108. Alternatively, the shaft 146 may be coupled to another portion or section of the tool body 140 that transitions from the first end 142 to the shaft 146.

In an exemplary embodiment, the tool body 140 includes a needle 160 at the working end 144 of the tool 108. The needle 160 is coupled to the shaft 146. For example, the needle 160 extends from the end of the shaft 146 to the working end 144. The needle 160 includes a tip 162 at the end of the tool 108. The tip 162 of the needle 160 may be used for piercing the tissues of the patient and/or for taking a biopsy of the tissues of the patient. In various embodiments, the needle 160 is hollow and open at the tip 162 to form a lumen, such as for taking a biopsy. In other various embodiments, the tip 162 of the needle 160 is closed. Other types of medical devices may be provided at the working end 144 of the tool 108 rather than the needle 160 for performing other types of medical procedures. The pressure sensor module 200 is used to take pressure measurements of the tissues proximate to the tip 162 of the needle 160.

In an exemplary embodiment, the needle 160 is separate and discrete from the shaft 146 and coupled to the end of the shaft 146 to form the working end 144 of the tool 108. For example, the needle 160 may be laser welded to the shaft 146 or secured to the shaft 146 using adhesive. The needle 160 is an extension of the shaft 146 and the body of the needle 160 forms part of the tool body 140.

In an exemplary embodiment, the tool body 140 is hollow having a tool channel 148 extending along at least a portion of the tool body 140. The tool channel 148 extends along the shaft 146. The tool channel 148 may additionally extend along the needle 160. An end of the needle 160 is received in the hollow interior of the shaft 146. Optionally, the needle 160 and the tool body 140 may have the same outer diameter. In an exemplary embodiment, the pressure sensor module 200 is received in the tool channel 148, such as immediately upstream of the needle 160. In alternative embodiments, the pressure sensor module 200 may be received in the needle 160. In an exemplary embodiment, the needle 160 is manufactured from a medical grade material, such as stainless steel. The tool body 140 is also manufactured from a medical grade material. The tool body 140 may be manufactured from a different material than the needle 160.

Figure 3 is a perspective view of the pressure sensor module 200 in accordance with an exemplary embodiment. The pressure sensor module 200 includes a pressure sensor 202, wires 204 coupled to and extending from the pressure sensor 202, and a carrier 206 used to hold the pressure sensor 202 and the wires 204. The carrier 206 is configured to be received in the tool body 140, such as in the shaft 146 (shown in Figure 2), to position the pressure sensor 202 relative to the tool body 140 for measuring pressure of fluid surrounding the tool 108.

The carrier 206 extends between a front 210 and a rear 212. In an exemplary embodiment, the carrier 206 is manufactured from a medical grade plastic material and manufactured by an injection molding process. However, in alternative embodiments, the carrier 206 may be manufactured from a metal material, such as stainless steel. The carrier 206 is sized and shaped to fit in the tool channel 148 of the tool body 140. The wires 204 extend from the rear 212 of the carrier 206. Optionally, the pressure sensor 202 may be located near the front 210 of the carrier 206. The pressure sensor 202 may be at other locations in alternative embodiments. Optionally, the carrier 206 may hold multiple pressure sensors 202, such as at spaced apart positions relative to each other. A wall or dam may be provided between the pressure sensors 202 to isolate the pressure readings by the pressure sensors. In other various embodiments, multiple carriers 206 may be provided at spaced apart locations each having a respective pressure sensor(s) 202 for taking pressure readings at different locations, such as in different tissues.

In an exemplary embodiment, the carrier 206 includes a chamber 214 that receives the pressure sensor 202. The chamber 214 may be open at a top 216 and/or a bottom 218 of the carrier 206. For example, the carrier 206 includes a top opening 220 and a bottom opening 222. The openings 220, 222 allow fluid flow into the chamber 214. In an exemplary embodiment, the pressure sensor 202 is aligned with the openings 220, 222. Additionally or alternatively, the chamber 214 may be open at the front 210 and/or the rear 212. The wires 204 extend into the chamber 214 for electrical connection to the pressure sensor 202. In an exemplary embodiment, a portion of the chamber 214 may be filled with potting material or epoxy to close off the chamber 214. The potting material or epoxy may surround the wires 204 and/or a portion of the pressure sensor 202 to provide strain relief for the wires 204.

In an exemplary embodiment, the carrier 206 includes sensor locating features 224 extending into the chamber 214 from a first side wall 226 and a second side wall 228. The sensor locating features 224 are used to position the pressure sensor 202 within the chamber 214. In various embodiments, the sensor locating features 224 may be crush ribs. The sensor locating features 224 center of the pressure sensor 202 in the chamber 214 between the first side wall 226 and the second side wall 228. The side walls 226, 228 flank the pressure sensor 202 to protect the pressure sensor 202, such as during shipping and handling as well as during use during the medical procedure.

In an exemplary embodiment, the carrier 206 includes one or more locating features 230 configured to engage the tool body 140 to locate the pressure sensor module 200 relative to the tool body 140. In the illustrated embodiment, the locating features 230 are locating tabs extending outward from the exterior of the carrier 206 at the front 210 of the carrier 206. For example, the locating features 230 are located on the first side wall 226 and the second side wall 228. The locating features 230 may be at other locations in alternative embodiments. Figure 2 illustrates the locating features 230 received in locating slot 158 of the tool body 140. The locating features 230 are configured to engage the tool body 140 to axially and rotatably position the pressure sensor module 200 relative to the tool body 140. Optionally, the locating features 230 may be keyed to orient the pressure sensor module 200 relative to the tool body 140. For example, the locating features 230 may be staggered or offset relative to each other on the opposite sides of the carrier 206. Other types of locating features may be used in alternative embodiments, such as rails, shoulders, grooves, openings or other types of locating features.

Figure 4 is a cross-sectional view of a portion of the tool 108 in accordance with an exemplary embodiment. Figure 4 illustrates the pressure sensor module 200 and the needle 160 coupled to the shaft 146 of the tool body 140. The pressure sensor module 200 is received in the tool channel 148 of the shaft 146. In an exemplary embodiment, the tool channel 148 is cylindrical to receive the cylindrical carrier 206. The carrier 206 positions the pressure sensor 202 within the tool channel 148. The wires 204 extend from the carrier 206 through the tool channel 148. The needle 160 is coupled to the end of the shaft 146 and extends forward of the shaft 146. Optionally, the needle 160 engages the front 210 of the carrier 206 to hold the carrier 206 in the tool channel 148.

In an exemplary embodiment, the shaft 146 of the tool body 140 includes fluid openings 150 that provide access to the tool channel 148. In the illustrated embodiment, the fluid openings 150 are provided at a top 152 and a bottom 154 of the tool body 140. The fluid openings 150 may be at other locations in alternative embodiments. When assembled, the fluid openings 150 are aligned with the chamber 214 of the carrier 206. The fluid openings 150 allow fluid flow from the exterior of the tool body 140 into the chamber 214 for pressure monitoring of the tissue fluid by the pressure sensor 202. The pressure sensor 202 is located in an interior of the tool body 140 and takes internal fluid pressure readings. In an exemplary embodiment, multiple fluid openings 150 are provided to allow fluid flow through the chamber 214. Alternatively, a single fluid opening 150 may be provided.

In an exemplary embodiment, the shaft 146 of the tool body 140 includes one or more sealing openings 156 that provide access to the tool channel 148. The sealing openings 156 may receive the potting material or the epoxy to seal the tool channel 148. For example, after the pressure sensor module 200 is assembled and positioned in the tool channel 148, the potting material or epoxy is injected into the tool channel 148 through the sealing openings 156. The potting material or epoxy may partially fill the chamber 214, such as the rear section of the chamber around a portion of the pressure sensor 202 and the wires 204. The potting material or epoxy may partially fill the tool channel 148 rearward of the carrier 206, such as to surround the portions of the wires 204 extending rearward from the carrier 206.

The pressure sensor 202 includes a strain gauge diaphragm 250 having conductive sensing elements 252 on the strain gauge diaphragm 250. The wires 204 are electrically connected to the conductive sensing elements 252. The pressure sensor 202 may be an integrated circuit component. For example, the strain gauge diaphragm 250 may be a silicone chip having the conductive sensing elements 252 fabricated on the silicone chip. In an exemplary embodiment, the pressure sensor 202 is a microelectromechanical systems (MEMS) device, such as a piezoresistive pressure sensor. Changes in the resistance of the conductive sensing elements 252 provide a measure of the pressure applied to the strain gauge diaphragm 250. The change in resistance may be proportional to the strain, which is the relative change in length of the conductive sensing element 252. In alternative embodiments, the pressure sensor 202 is a capacitive pressure sensor. Deformation of the strain gauge diaphragm 250 changes the spacing between the conductive sensing elements 252, which causes changes in capacitance of the circuit. The change in capacitance can be measured by frequency changes within the circuit, which corresponded to changes in pressure.

The strain gauge diaphragm 250 is coupled to the carrier 206. For example, the strain gauge diaphragm 250 is cantilevered forward of a support wall 232 of the carrier 206 into the chamber 214. The carrier 206 provides protection for the strain gauge diaphragm 250. The support wall 232 supports the rear end of the strain gauge diaphragm 250. The support wall 232 supports the wires 204. A front portion of the strain gauge diaphragm 250 extends forward of the support wall 232 and is unsupported within the chamber 214. The chamber 214 is open above, below, and on the sides of the strain gauge diaphragm 250 to allow the pressure sensor 202 to function properly. For example, the front portion of the strain gauge diaphragm 250 is able to flex, bend, oscillate or otherwise move within the chamber 214 as the pressure of the fluid in the tissues changes.

In an exemplary embodiment, pressure changes may be monitored in real time as the tool 108 moves through the body of the patient. The pressure of the fluid may change as the tool 108 moves through different tissues in the body of the patient. The pressure sensor 202 is located proximate to the tip 162 of the needle 160 to measure the pressure of the fluid within the tissue that is in the vicinity of the tip 162 of the needle 160. The pressure readings on the pressure sensor 202 may be used to aid the physician in performing the medical procedure, such as for guiding the needle 160 through the various tissues. For example, the pressure readings (for example, by identifying changes in pressure) may indicate when the needle 160 is located in certain tissues, at a tissue wall, in damaged tissue, and the like.

In an exemplary embodiment, the tool 108 is modular and may have different diameters. The carrier 206 may have different diameters to correspond with the different diameter tool bodies 140. In various embodiments, the carrier 206 has a color corresponding to a particular diameter. In an exemplary embodiment, the carrier 206 is manufactured from a material having a coefficient of thermal expansion that is similar to the coefficient of thermal expansion of the tool body 140. The carrier 206 is manufactured from a material having sufficient elasticity to allow the carrier 206 to flex through the required bend radius. In various embodiments, the carrier 206 may be manufactured from a transparent or translucent material. The pressure sensor module 200 may include a lighting element (not shown) transmitting light when the pressure sensor module 200 is operating. The light from the lighting element passes through the carrier 206. The lighting element may be an LED. The lighting element may be a light pipe or an optical fiber transmitting light.

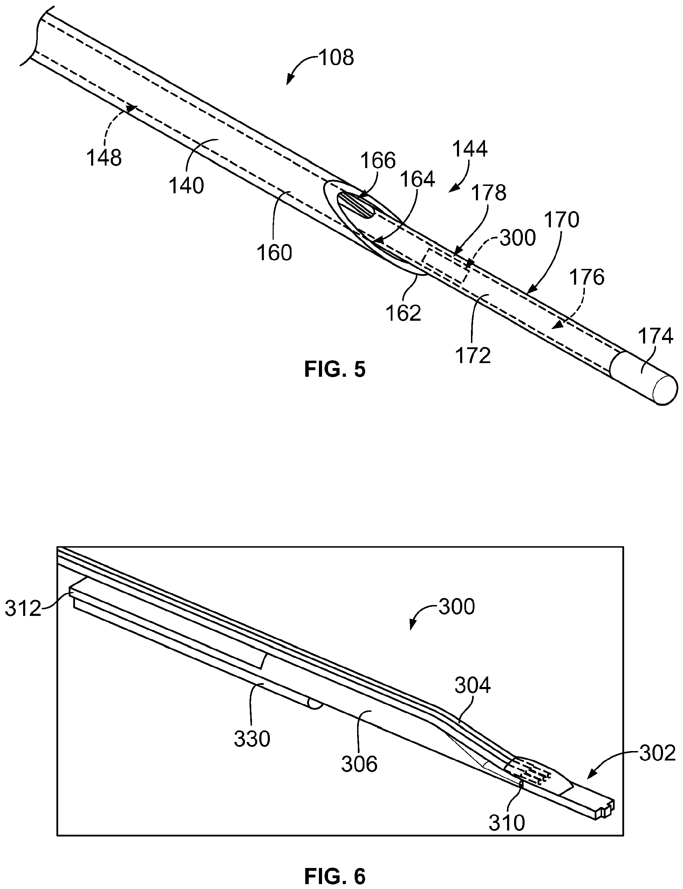

Figure 5 is a perspective view of the tool 108 in accordance with an exemplary embodiment. The tool 108 includes the tool body 140 extending to the second end 144 of the tool 108. Figure 5 illustrates the needle 160 of the tool 108. The needle 160 defines the tool body 140 at the second end 144. In an exemplary embodiment, the needle 160 includes a lumen 164 forming a portion of the tool channel 148. The needle 160 includes a lumen port 166 open at the second end 144 to provide access to the lumen 164. The needle 160 may be used to take a biopsy of a tissue of the patient.

In an exemplary embodiment, the tool 108 includes a stylet 170 or slender probe received in the lumen 164. The stylet 170 is used to plug the lumen 164. The stylet is movable relative to the needle 160. The stylet 170 is movable to an extended position (shown in Figure 5) and a retracted position. The stylet 170 includes an inner shaft 172 and a plunger 174 at a front of the inner shaft 172. The plunger 174 and a portion of the inner shaft 172 is exposed beyond the tip 162 of the needle 160 in the extended position. In the retracted position, the plunger 174 may be at the tip 162 or may be recessed rearward of the tip 162 or may be slightly extended forward of the tip 162. In an exemplary embodiment, the inner shaft 172 is manufactured from a medical grade material, such as stainless steel. The plunger 174 is also manufactured from a medical grade material, which may be a different material than the inner shaft 172. The plunger may be welded to the inner shaft 172 or secured using adhesive. In alternative embodiments, the plunger 174 is integral with the inner shaft 172.

In an exemplary embodiment, the stylet 170 holds the pressure sensor module 300. For example, the pressure sensor module 300 is held in an interior channel 176 of the inner shaft 172. The stylet 170 includes one or more fluid openings 178 that provide access to the channel 176. In the illustrated embodiment, the fluid openings 178 are provided at the top and the bottom of the inner shaft 172. The fluid openings 178 may be at other locations in alternative embodiments. The fluid openings 178 may be aligned with the pressure sensor module 300 to allow fluid flow from the exterior of the stylet 170 into the interior channel 176 for pressure monitoring of the tissue fluid by the pressure sensor module 300. The stylet 170 may be moved to the extended position to position the pressure sensor module 300 proximate to the tip 162 of the needle 160.

Figure 6 is a perspective view of the pressure sensor module 300 in accordance with an exemplary embodiment. The pressure sensor 302 is similar to the pressure sensor 202 (shown in Figure 3). The pressure sensor module 300 includes a pressure sensor 302, wires 304 coupled to and extending from the pressure sensor 302, and a carrier 306 used to hold the pressure sensor 302 and the wires 304. The carrier 306 is shaped differently than the carrier 206 (shown in Figure 3).

The carrier 306 extends between a front 310 and a rear 312. In an exemplary embodiment, the carrier 306 is manufactured from a medical grade plastic material and manufactured by an injection molding process. However, in alternative embodiments, the carrier 306 may be manufactured from a metal material, such as stainless steel. The wires 304 extend from the rear 312 of the carrier 306. Optionally, the pressure sensor 302 may be located near the front 310 of the carrier 306. The pressure sensor 302 may be at other locations in alternative embodiments. Optionally, the carrier 306 may hold multiple pressure sensors 302, such as at spaced apart positions relative to each other. A wall or dam may be provided between the pressure sensors 302 to isolate the pressure readings by the pressure sensors. In other various embodiments, multiple carriers 306 may be provided at spaced apart locations each having a respective pressure sensor(s) 302 for taking pressure readings at different locations, such as in different tissues.

In an exemplary embodiment, the carrier 306 includes one or more locating features 330 configured to engage the tool body 140 to locate the pressure sensor module 300 relative to the tool body 140. In the illustrated embodiment, the locating feature 330 is a locating tab extending outward from the exterior of the carrier 306 at the bottom of the carrier 306. Other types of locating features may be used in alternative embodiments, such as rails, shoulders, grooves, openings or other types of locating features.

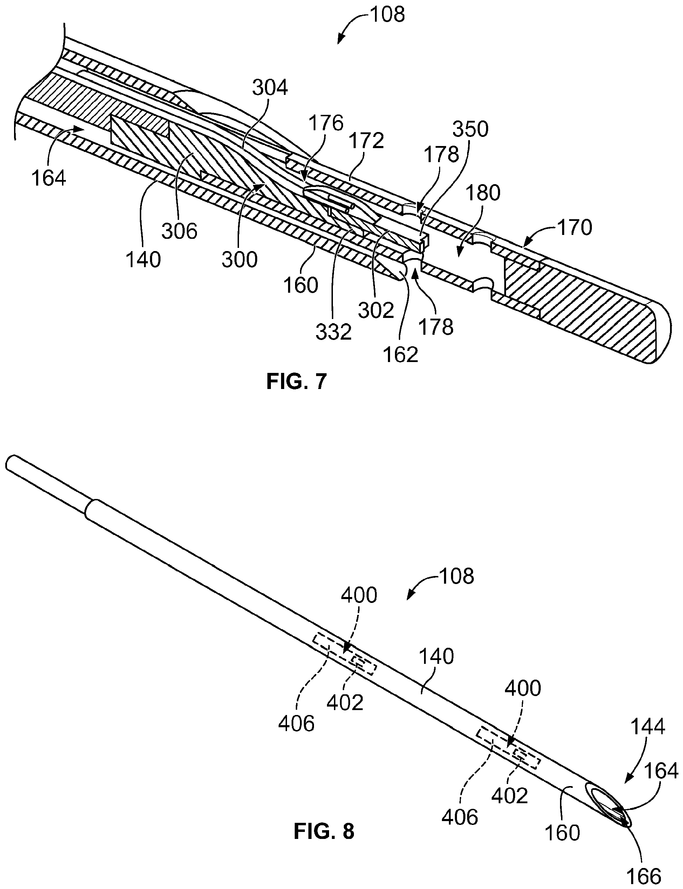

Figure 7 is a cross-sectional view of a portion of the tool 108 in accordance with an exemplary embodiment. Figure 7 illustrates the pressure sensor module 300 received in the interior channel 176 of the stylet 170 and coupled to the inner shaft 172. The stylet 170 and the pressure sensor module 300 are located in the lumen 164 of the needle 160. In an exemplary embodiment, the interior channel 176 is cylindrical to receive the carrier 306. The carrier 306 positions the pressure sensor 302 within the interior channel 176. The wires 304 extend from the pressure sensor 302 along the top of the carrier 306.

In an exemplary embodiment, the stylet 170 includes a chamber 180 that receives the pressure sensor 302. The chamber 180 is defined by the inner shaft 172 and the carrier 306. The chamber 180 is open above, below and along the sides of the pressure sensor 302. The fluid openings 178 are open to the chamber 180. In an exemplary embodiment, a portion of the chamber 180 may be filled with potting material or epoxy to close off the chamber 180 and provide strain relief for the wires 304. The fluid openings 178 allow fluid flow from the exterior of the tool 108 into the chamber 180 for pressure monitoring of the tissue fluid by the pressure sensor 302.

The strain gauge diaphragm 350 is coupled to the carrier 306. For example, the strain gauge diaphragm 350 is cantilevered from a support wall 332. The support wall 332 supports the rear end of the strain gauge diaphragm 350. A front portion of the strain gauge diaphragm 350 extends from the carrier 306 into the chamber 180. The strain gauge diaphragm 350 is unsupported within the chamber 180. The chamber 180 is open above, below, and on the sides of the strain gauge diaphragm 350 to allow the pressure sensor 302 to function properly. For example, the front portion of the strain gauge diaphragm 350 is able to flex, bend, oscillate or otherwise move within the chamber 180 as the pressure of the fluid in the tissues changes.

In an exemplary embodiment, the pressure sensor 302 is located proximate to the tip 162 of the needle 160 to measure the pressure of the fluid within the tissue that is in the vicinity of the tip 162 of the needle 160. The pressure sensor 302 is located exterior of the tool body 140 and takes external fluid pressure readings. The pressure readings on the pressure sensor 302 may be used to aid the physician in performing the medical procedure, such as for taking a biopsy. For example, the pressure sensor 302 may determine the pressure of the fluid in particular tissue and the physician is able to compare pressure readings in different tissues to determine if the tissue is healthy or damaged (damaged tissue may by harder and have higher fluid pressure).

Figure 8 is a perspective view of the tool 108 in accordance with an exemplary embodiment. The tool 108 includes the tool body 140 extending to the second end 144 of the tool 108. Figure 8 illustrates the needle 160 of the tool 108. The needle 160 includes the lumen 164 forming a portion of the tool channel 148. A pressure sensor module 400 is located in the lumen 164. The pressure sensor module 400 may be coupled to a stylet or coupled to the body of the needle 160.

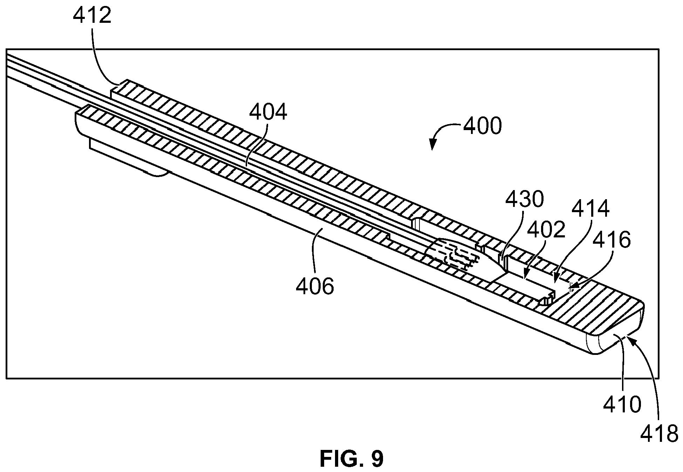

Figure 9 is a perspective view of the pressure sensor module 400 in accordance with an exemplary embodiment. The pressure sensor 402 is similar to the pressure sensor 202 (shown in Figure 4). The pressure sensor module 400 includes a pressure sensor 402, wires 404 coupled to and extending from the pressure sensor 402, and a carrier 406 used to hold the pressure sensor 402 and the wires 404. The carrier 406 is shaped differently than the carrier 206 (shown in Figure 4).

The carrier 406 extends between a front 410 and a rear 412. The carrier 406 includes a chamber 414 in a top 416 of the carrier 406. The chamber 414 receives the pressure sensor 402 and the wires 404. In the illustrated embodiment, the chamber 414 is open at the rear 412 and closed at the front 410. The wires 404 extend through the opening at the rear 412. The portion of the carrier 406 forward of the chamber 414 protects the pressure sensor 402. The portions of the carrier 406 along the sides of the chamber 414 protects the pressure sensor 402. Optionally, the carrier 406 may include a bottom channel 418 at the bottom that is in fluid communication with the chamber 414. The bottom channel 418 allows fluid flow into or out of the chamber 414. In the illustrated embodiment, the top 416 of the carrier 406 is flat, being formed by truncating a cylinder. When the carrier 406 is loaded into the tool body 140, the space above the carrier 406 is open allowing fluid flow into or out of the chamber 414 from above. In an exemplary embodiment, the carrier 406 is manufactured from a medical grade plastic material and manufactured by an injection molding process. However, in alternative embodiments, the carrier 406 may be manufactured from a metal material, such as stainless steel. Optionally, the pressure sensor 402 may be located near the front 410 of the carrier 406. The pressure sensor 402 may be at other locations in alternative embodiments. Optionally, the carrier 406 may hold multiple pressure sensors 402, such as at spaced apart positions relative to each other. A wall or dam may be provided between the pressure sensors 402 to isolate the pressure readings by the pressure sensors. In other various embodiments, multiple carriers 406 may be provided at spaced apart locations each having a respective pressure sensor(s) 402 for taking pressure readings at different locations, such as in different tissues.

In an exemplary embodiment, the carrier 406 includes one or more locating features 430 configured to engage the tool body 140, such as the needle 160 or the stylet (if used), to locate the pressure sensor module 400 relative to the tool body 140. In the illustrated embodiment, the locating feature 430 is a locating tab extending into the chamber 414 to engage the pressure sensor module 400 and locate the pressure sensor module 400 in the chamber 414. Optionally, multiple locating features 430 may be provided, such as at both sides of the chamber 414. The locating features 430 may form a pocket or well for receiving epoxy used to fix or secure the pressure sensor module 400 in the carrier 406. The locating features 430 prevent the epoxy from wicking into the distal portion of the pressure sensor 402, which prevents interference with operation of the pressure sensor 402. Other types of locating features may be used in alternative embodiments, such as rails, shoulders, grooves, openings or other types of locating features.

Returning to Figure 8, in the illustrated embodiment, the tool 108 includes two carriers 406 and corresponding pressure sensors 402 spaced apart from each other within the tool channel 148. The tool body 140 may include fluid openings aligned with the pressure sensors to allow fluid flow into the chambers 414 of the carriers 406. Alternatively, the chambers 414 are in fluid communication with the lumen port 166 at the front of the needle 160 to receive the fluid. The pair of pressure sensors 402 are both able to measure pressure of the fluid at two different, spaced apart locations. The pressure sensors are linearly offset from each other, such as one pressure sensor located distal of the other pressure sensor. The pressure sensor module 400 is able to measure a pressure difference between the two pressure readings, such as to measure pressure from different tissues. The tool 108 may be used to measure pressure difference of healthy tissue and damaged tissue. Optionally, the carriers 406 are spaced apart from each other. The pressure sensors 402 are physically separated from each other, such as by a wall such that the fluids surrounding the pressure sensors 402 do not comingle. In various embodiments, the carriers 406 may abut against each other within the tool channel 148. In other alternative embodiments, a single carrier 406 is provided holding the pair of pressure sensors 402 at spaced apart locations from each other. The single carrier 406 includes one or more walls forming a dam between the chambers 414 to isolate the fluid and allow the two pressure sensors 402 to measure the difference in fluid pressure between the fluids.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the invention without departing from its scope. Dimensions, types of materials, orientations of the various components, and the number and positions of the various components described herein are intended to define parameters of certain embodiments, and are by no means limiting and are merely exemplary embodiments. Many other embodiments and modifications within the scope of the claims will be apparent to those of skill in the art upon reviewing the above description. The scope of the invention should, therefore, be determined with reference to the appended claims. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. An interventional medical device (100) comprising:
an insertion tube (120) extending between a proximal end (102) and a distal end (104), the distal end (104) configured to be inserted into internal tissues of a patient during a medical procedure, the insertion tube (120) having an internal channel (122);
a tool (108) received in the internal channel (122) of the insertion tube (120), the tool (108) having a tool body (140) and a needle (160) at an end of the tool body (140) forming a tip (162) of the tool (108), the tool body (140) forming a tool channel (176); and
a pressure sensor module (200) received in the tool channel (176), the pressure sensor module (200) including a pressure sensor (202) and a carrier (206) holding the pressure sensor (202), the carrier (206) being coupled to the tool body (140) to position the pressure sensor (202) in the tool channel (176) proximate to the tip (162) of the tool (108) for measuring pressure of fluid of the internal tissues of the patient.

2. The interventional medical device (100) of claim 1, wherein the tool body (140) includes at least one opening (150) in flow communication with the pressure sensor (202), the opening (150) configured to allow fluid flow into the tool channel (176).

3. The interventional medical device (100) of claim 1 or 2, wherein the pressure sensor (202) includes a strain gauge diaphragm (250) coupled to the carrier (206) and conductive sensing elements (252) on the strain gauge diaphragm (250) for measuring changes in resistance to provide a measure of pressure applied to the strain gauge diaphragm (250).

4. The interventional medical device (100) of claim 1, 2 or 3 wherein the carrier (206) includes side walls (232) flanking the pressure sensor (202), the side walls (232) being located between the pressure sensor (202) and the tool body (140).

5. The interventional medical device (100) of any preceding claim, wherein the carrier (206) includes a chamber (214) configured to receive the fluid, the pressure sensor (202) extending into the chamber (214), preferably wherein the chamber (214) is open above, below and along sides of the pressure sensor (202).

6. The interventional medical device (100) of any preceding claim, wherein the carrier (206) includes a locating feature (230), the locating feature (230) engaging the tool body (140) to locate the pressure sensor module (200) in the internal channel (122).

7. The interventional medical device (100) of any preceding claim, wherein the carrier (206) includes an exterior surface having a curved profile having a radius of curvature corresponding to a radius of curvature of the tool body (140).

8. The interventional medical device (100) of any preceding claim, wherein the carrier (206) is either transparent or translucent, the pressure sensor module (200) including a lighting element transmitting light when operating, the light passing through the carrier (206).

9. The interventional medical device (100) of any preceding claim, wherein the pressure sensor module (200) includes wires (204) coupled to the pressure sensor (202), the carrier (206) supporting ends of the wires (204) relative to the pressure sensors (202), the wires (204) routed through the internal channel (122) to the proximal end (102) of the insertion tube (120).

10. The interventional medical device (100) of any preceding claim, wherein the needle (160) includes a lumen (164), the pressure sensor module (300) located within the lumen (164), preferably (i) wherein the tool (108) includes a stylet (170) received in the tool channel (148), the stylet (170) being extendable from the end of the needle (160), the pressure sensor module (300) coupled to, and movable with, the stylet (170), wherein the pressure sensor (302) is configured to be located exteriorly of the tool body (140) with the stylet (170) when the stylet (170) is extended, the pressure sensor (302) being located interiorly of the tool body (140) with the stylet (170) when the stylet (170) is retracted into the needle (160), or (ii) wherein the lumen (164) includes a lumen port (166) at the end of the needle (160), the lumen port (166) being open to allow the fluid to flow into the lumen (164), the pressure sensor (302) positioned upstream of the lumen port (166) to measure the pressure of the fluid inside the lumen (164).

11. The interventional medical device (100) of any preceding claim, wherein the carrier (306) includes a front (310) and a rear (312), the pressure sensor (302) extending forward of the front (310) of the carrier (306).

12. The interventional medical device (100) of any preceding claim, wherein the pressure sensor (402) is a first pressure sensor, the pressure sensor module (400) including a second pressure sensor (402) remote from the first pressure sensor (402), the pressure sensor module determining a pressure difference between the first pressure sensor (402) and the second pressure sensor (402).

13. The interventional medical device (100) of any preceding claim, further comprising:
a handle (106), the proximal end (102) of the insertion tube (120) extending from the handle (106);
a light transmitter (116) received in the internal channel (122) of the insertion tube (120) and extending to the distal end (104) for illuminating the internal tissues of the patient; and
a camera (112) at the distal end (104) for imaging the internal tissues of the patient, the camera (112) having a cable extending form the camera (112) through the internal channel (122).

14. The interventional medical device (100) of any preceding claim, wherein the pressure sensor module (400) includes a first pressure sensor (402) and a second pressure sensor (402) remote from the first pressure sensor (402), the first and second pressure sensors (402) configured to measure pressure of fluid in the internal tissues, the pressure sensor module (400) determining a first pressure from the first pressure sensor (402) and determining a second pressure from the second pressure sensor (402), preferably wherein the carrier (406) includes a front and a rear, the first pressure sensor (402) is located proximate to the front, the second pressure sensor (402) is located proximate to the rear.
